# EUROPEAN PATENT APPLICATION

(11) **EP 3 369 360 A1**
(43) Date of publication of application: **05.09.2018**
(21) Application number: 15907226.3
(22) Date of filing: 27.10.2015
(51) Int. Cl.: A61B 1/04

(54) **IMAGE PICKUP UNIT AND ENDOSCOPE**

(71) Applicant: Olympus Corporation, Hachioji-shi, Tokyo 192-8507 (JP)
(72) Inventor: IGARASHI, Takatoshi, Tokyo 192-8507 (JP)
(74) Representative: Winter, Brandl, Fürniss, Hübner, Röss, Kaiser, Polte - Partnerschaft mbB
(86) International application number: PCT/JP2015/080277
(87) International publication number: WO 2017/072862

(57) **Abstract**

An imaging unit and an endoscope, which enable further downsizing to be realized and are high in reliability, are provided. An imaging unit 40 includes: an imaging chip 43 that generates an image signal by receiving light via a light receiving surface thereof and performing photoelectric conversion; at least one semiconductor chip 44 having a size fitting into a plane of optical axis direction projection of the imaging chip 43, the size being projected onto a plane orthogonal to an optical axis direction; and a reinforcement member 46 that is arranged on at least one side surface of the semiconductor chip 44; the semiconductor chip 44 is layered and mounted on a back side of the light receiving surface of the imaging chip 43; and the reinforcement member 4 is arranged to cover a connection interface between the imaging chip 43 and the semiconductor chip 44, or between the semiconductor chips 44.

## Description

### Field

The present invention relates to: an imaging unit that is inserted in a subject, captures an image inside the subject, and generates data of the image therein; and an endoscope including the imaging unit at a distal end portion thereof.

### Background

Conventionally, endoscopes acquire in-vivo images of subjects, such as patients, by flexible insertion portions thereof being inserted in the subjects, the flexible insertion portions having imaging devices provided at distal ends thereof and being elongated. An imaging unit used in such an endoscope includes: a semiconductor chip having an imaging element formed therein; and a circuit board arranged adjacently to a back side of this semiconductor chip, and mounted with electronic components, such as a condenser, a resistance, and an IC chip, which form a drive circuit of the imaging element (see, for example, Patent Literature 1).

### Citation List

### Patent Literature

Patent Literature 1: Japanese Patent No. 4589659 Summary

### Technical Problem

According to the above cited Patent Literature 1, since the electronic components, such as the condenser, the resistance, and the IC chip, are mounted on the circuit board connected to a back surface of the imaging element, a rigid portion of the imaging unit is increased in length in an optical axis direction thereof. Use of such an imaging unit in an endoscope may be a burden on patients. For the purpose of relieving the burden on patients, there has been a demand for a small sized imaging unit having a rigid portion decreased in length with its radial direction size being kept small.

In recent years, due to development of semiconductor chips having planar devices formed therein, by semiconductor chips being layered over one another and being mounted on a back surface of an imaging element, reduction in length of its rigid portion has been attempted. However, when plural semiconductor chips are layered over one another and connected, connected portions are decreased in durability against force in a shearing direction (a direction orthogonal to a layering direction). Therefore, if stress is exerted on the connected portions in a process where the imaging device is assembled with the distal end portion of the endoscope, reliability of the imaging device may be reduced, and the yield may be reduced due to breakage thereof.

The present invention has been made in view of the above, and an object thereof is to provide an imaging unit and an endoscope that enable downsizing and that are highly reliable.

### Solution to Problem

To solve the above-described problem and achieve the object, an imaging unit according to the present invention includes: an imaging chip configured to generate an image signal by receiving light via a light receiving surface thereof and performing photoelectric conversion; at least one semiconductor chip having a size fitting into a plane of projection of the imaging chip, the size being projected onto a plane orthogonal to an optical axis direction; and a reinforcement member arranged on at least one side surface of the semiconductor chip, wherein the semiconductor chip is layered and mounted on a back side of the light receiving surface of the imaging chip, and the reinforcement member is arranged to cover a connection interface between the imaging chip and the semiconductor chip, or a connection interface between the semiconductor chips.

Moreover, in the above-described imaging unit according to the present invention, the reinforcement member has a cross section having a shape of an L-shaped plate, the cross section being orthogonal to the optical axis direction, and the reinforcement member is arranged to cover two sides of the semiconductor chip, or two sides of the imaging chip and the semiconductor chip.

Moreover, in the above-described imaging unit according to the present invention, the reinforcement member is cylindrical, and is arranged on an outer peripheral side surface of the semiconductor chip, or an outer peripheral side surface of the imaging chip and the semiconductor chip.

Moreover, in the above-described imaging unit according to the present invention, the imaging chip includes a first stepped portion on a back side of the light receiving surface, and the reinforcement member is cylindrical, and is arranged on an outer peripheral side surface of the first stepped portion and the semiconductor chip.

Moreover, in the above-described imaging unit according to the present invention, a size of the reinforcement member projected onto the plane orthogonal to the optical axis direction is a size fitting into the plane of optical axis direction projection of the imaging chip.

Moreover, the above-described imaging unit according to the present invention includes a cover glass configured to cover a light receiving unit of the imaging chip, wherein the cover glass includes a second stepped portion on a surface thereof that contacts the imaging chip, and the reinforcement member is cylindrical, and is arranged on an outer peripheral side surface of the second stepped portion, the imaging chip, and the semiconductor chip/chips.

Moreover, in the above-described imaging unit according to the present invention, a size of the reinforcement member projected onto the plane orthogonal to the optical axis direction is a size fitting into a plane of optical axis direction projection of the cover glass.

Moreover, in the above-described imaging unit according to the present invention, the imaging chip and the semiconductor chip are electrically and mechanically connected to each other by through-vias formed respectively in the imaging chip and the semiconductor chip.

Moreover, an endoscope according to the present invention includes: any one of the above-described imaging units; and an insertion portion including a distal end portion and being insertable in a subject, the distal end portion being formed of a rigid member and being cylindrical, wherein the insertion portion includes the imaging unit in an inner space of the distal end portion. Advantageous Effects of Invention

The present invention has an effect of enabling provision of an imaging unit and an endoscope, which are highly reliable and are able to be downsized.

### Brief Description of Drawings

FIG. 1 is a diagram schematically illustrating an overall configuration of an endoscope system according to a first embodiment of the present invention.
FIG. 2 is a partial sectional view of a distal end portion of an endoscope according to the first embodiment of the present invention.
FIG. 3A is a sectional view of an imaging unit according to the first embodiment of the present invention.
FIG. 3B is a rear view of the imaging unit according to the first embodiment of the present invention.
FIG. 4A is a sectional view of an imaging unit according to a first modified example of the first embodiment of the present invention.
FIG. 4B is a rear view of the imaging unit according to the first modified example of the first embodiment of the present invention.
FIG. 5A is a rear view of an imaging unit according to a second modified example of the first embodiment of the present invention.
FIG. 5B is a rear view of an imaging unit according to a third modified example of the first embodiment of the present invention.
FIG. 5C is a rear view of an imaging unit according to a fourth modified example of the first embodiment of the present invention.
FIG. 5D is a rear view of an imaging unit according to a fifth modified example of the first embodiment of the present invention.
FIG. 5E is a rear view of an imaging unit according to a sixth modified example of the first embodiment of the present invention.
FIG. 6 is a sectional view of an imaging unit according to a seventh modified example of the first embodiment of the present invention.
FIG. 7 is a sectional view of an imaging unit according to an eighth modified example of the first embodiment of the present invention.
FIG. 8 is a sectional view of an imaging unit according to a second embodiment of the present invention.
FIG. 9 is a sectional view of an imaging unit according to a first modified example of the second embodiment of the present invention.
FIG. 10 is a sectional view of an imaging unit according to a third embodiment of the present invention.
FIG. 11 is a sectional view of an imaging unit according to a first modified example of the third embodiment of the present invention.
FIG. 12 is a sectional view of an imaging unit according to a second modified example of the third embodiment of the present invention.
FIG. 13 is a sectional view of an imaging unit according to a third modified example of the third embodiment of the present invention.

### Description of Embodiments

Hereinafter, as modes for carrying out the present invention (hereinafter, referred to as "embodiments"), endoscopes having imaging devices will be described. Further, the present invention is not limited by these embodiments. Furthermore, each drawing referred to in the following description schematically illustrates shapes, sizes, and positional relations merely to an extent that allows contents of the present invention to be understood. That is, the present invention is not limited only to the shapes, sizes, and positional relations illustrated as examples in the drawings. Moreover, portions that differ in their dimensions and ratios among the drawings may be included, too.

### (First Embodiment)

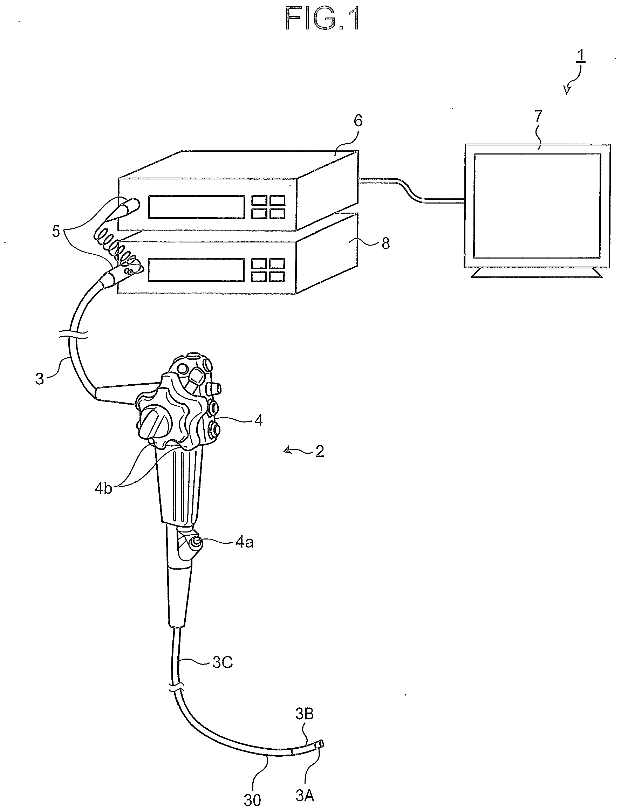

FIG. 1 is a diagram schematically illustrating an overall configuration of an endoscope system according to a first embodiment of the present invention. An endoscope system 1 illustrated in FIG. 1 includes an endoscope 2, a universal cord 3 (a transmission cable), a connector portion 5, a processor 6 (a control device), a display device 7, and a light source device 8.

By an insertion portion 30 being inserted in a subject, the endoscope 2 captures an in-vivo image of the subject, and outputs an image signal (image data) to the processor 6. A bundle of electric cables inside the universal cord 3 is extended to the insertion portion 30 of the endoscope 2, and connects to an imaging device provided in a distal end portion 3A of the insertion portion 30. An operating unit 4, which is provided with various buttons and knobs for operation of endoscopic functions, is connected at a proximal end side of the insertion portion 30 of the endoscope 2. The operating unit 4 has a treatment tool insertion opening 4a, through which treatment tools, such as biopsy forceps, an electric knife, and an inspecting probe, are inserted in a body cavity of the subject.

The connector portion 5 is provided at a proximal end of the universal cord 3, and is connected to the processor 6 and the light source device 8. The connector portion 5 executes predetermined signal processing on an image signal output by the imaging device in the distal end portion 3A connected to the universal cord 3, and outputs a digital image signal to the processor 6 by executing A/D conversion on this image signal.

The processor 6 executes predetermined image processing on the image signal output from the connector portion 5, and outputs this image signal to the display device 7. Further, the processor 6 controls the whole endoscope system 1. The processor 6 is configured by use of a central processing unit (CPU) or the like.

The display device 7 displays thereon an image corresponding to the image signal output from the processor 6. The display device 7 is configured by use of, for example, a display panel of liquid crystal or organic electro luminescence (EL).

The light source device 8 emits illumination light to the subject from a distal end of the insertion portion 30 of the endoscope 2 via the connector portion 5 and the universal cord 3. The light source device 8 is configured by use of a xenon lamp, a light emitting diode (LED) lamp, or the like.

The insertion portion 30 has: the distal end portion 3A having the imaging device provided therein; a bending portion 3B that is provided connectively to a proximal end side of the distal end portion 3A and that is freely bendable in plural directions; and a flexible tube portion 3C that is provided connectively to a proximal end side of this bending portion 3B. The image signal captured by the imaging device provided in the distal end portion 3A is connected to the connector portion 5 via the operating unit 4 by the universal cord 3 having a length of, for example, several meters. The bending portion 3B is bent by operation on a bending operation knob 4b provided in the operating unit 4, and is freely bendable in four directions, for example, upward, downward, leftward, and rightward, in association with pulling and loosening of a bending wire inserted through the insertion portion 30.

Further, the endoscope 2 has a light guide (not illustrated in the drawings) provided therein for propagation of illumination light from the light source device 8, and has an illumination lens (not illustrated in the drawings) provided at an illumination light emission end of this light guide. This illumination lens is provided at the distal end portion 3A of the insertion portion 30.

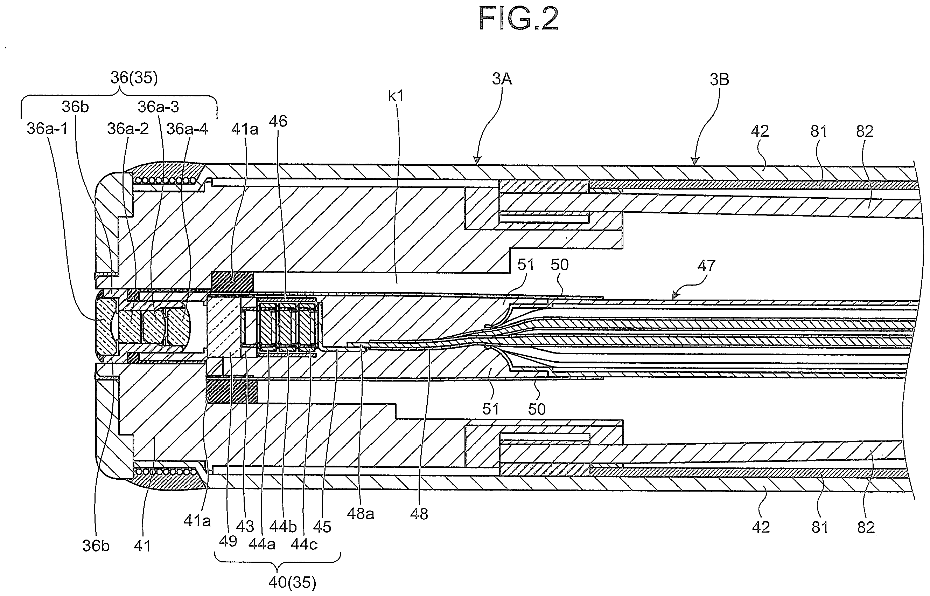

Next, a configuration of the distal end portion 3A of the endoscope 2 will be described in detail. FIG. 2 is a partial sectional view of the distal end portion 3A of the endoscope 2, and illustrates a partial cross section obtained when the distal end portion 3A is cut along a plane orthogonal to a board surface of the imaging device provided in the distal end portion 3A of the endoscope 2, the plane being parallel to an optical axis direction of the imaging device. Further, FIG. 2 illustrates the distal end portion 3A and a part of the bending portion 3B, of the insertion portion 30 of the endoscope 2.

As illustrated in FIG. 2, the bending portion 3B is freely bendable in four directions, upward, downward, leftward, and rightward, in association with pulling and loosening of a bending wire 82 inserted through a bending tube 81 provided inside a covering tube 42 described later. An imaging device 35 is provided inside the distal end portion 3A extendedly provided at a distal end side of this bending portion 3B.

The imaging device 35 has a lens unit 36, and an imaging unit 40 arranged at a proximal end side of the lens unit 36. The imaging device 35 is adhered to an inner side of a distal end portion main body 41 by an adhesive 41a. The distal end portion main body 41 is cylindrically formed of a rigid member or the like for formation of an inner space k1 accommodating therein the imaging device 35. A proximal end side outer peripheral portion of the distal end portion main body 41 is covered by the covering tube 42 that is flexible. A member on a proximal end side of the distal end portion main body 41 is formed of a flexible member such that the bending portion 3B is bendable. The distal end portion 3A where the distal end portion main body 41 is arranged serves as a rigid portion of the insertion portion 30.

The lens unit 36 has plural objective lenses 36a-1 to 36a-4, and a lens holder 36b that holds the plural objective lenses 36a-1 to 36a-4. By a distal end of the lens holder 36b being fixed by being inserted into the distal end portion main body 41, the lens unit 36 is fixed to the distal end portion main body 41. The plural objective lenses 36a-1 to 36a-4 form a subject image.

The imaging unit 40 includes: an imaging chip 43 having a light receiving unit that generates an electric signal (an image signal) by receiving light and executing photoelectric conversion, like a charge coupled device (CCD) or a complementary metal oxide semiconductor (CMOS); semiconductor chips 44a, 44b, and 44c that are layered over one another and mounted on a back side of a light receiving surface of the imaging chip 43 and have planar devices formed therein; a flexible printed circuit board 45 (hereinafter, referred to as the "FPC board 45") that extends in the optical axis direction from the semiconductor chip 44c; various signal cables 48 that are mounted on a surface of the FPC board 45 and transmit electric signals (image signals) or the like; and a cover glass 49 that adheres to the imaging chip 43 in a state of covering the light receiving unit of the imaging chip 43. A detailed configuration of the imaging unit 40 will be described later.

Proximal ends of the signal cables 48 extend toward a proximal end of the insertion portion 30. An electric cable bundle 47 that is a bundle of the signal cables 48 is arranged in the distal end portion main body 41 to be able to be inserted through the insertion portion 30, and is provided extendedly to the connector portion 5 via the operating unit 4 and the universal cord 3, which are illustrated in FIG. 1.

The subject image formed by the objective lenses 36a-1 to 36a-4 of the lens unit 36 is converted to an image signal by being received and photoelectrically converted by the imaging chip 43 arranged at an image forming position of the objective lenses 36a-1 to 36a-4. The image signal generated by the imaging chip 43 is output to the processor 6, via the semiconductor chips 44a, 44b, and 44c, the FPC board 45, the signal cables 48 connecting to the FPC board, and the connector portion 5.

Outer peripheries of the imaging device 35 and a distal end portion of the electric cable bundle 47 are covered by a heat shrinkable tube 50, for improvement of their durability. Gaps among parts inside the heat shrinkable tube 50 are filled with a sealing resin 51.

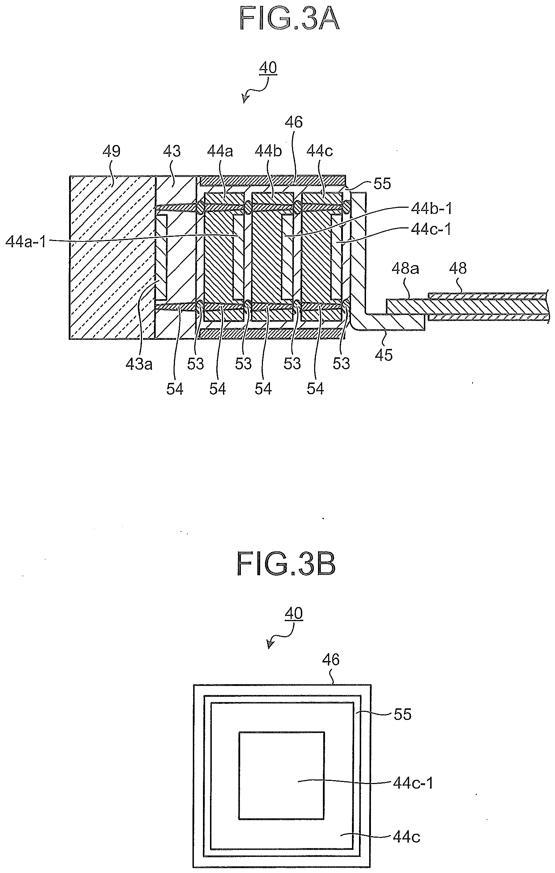

Next, the imaging unit 40 will be described in detail. FIG. 3A is a sectional view of the imaging unit 40. FIG. 3B is a rear view of the imaging unit 40, and illustration of the FPC board 45 is omitted therein for understanding of the invention. The imaging unit 40 illustrated in FIG. 3 includes the above described cover glass 49, imaging chip 43, semiconductor chips 44a, 44b, and 44c, and FPC board 45, and a reinforcement member 46.

The imaging chip 43 has: a light receiving unit 43a that generates an image signal by receiving and performing photoelectric conversion on a subject image formed by the objective lenses 36a-1 to 36a-4 of the lens unit 36; and a through-via 54 (a through-silicon via: TSV) that propagates therethrough the image signal generated by the light receiving unit 43a; and the through-via 54 formed in the imaging chip 43 is connected to the semiconductor chip 44a via a bump 53.

The semiconductor chips 44a, 44b, and 44c respectively have planar devices 44a-1, 44b-1, and 44c-1, formed therein, and are layered over one another and mounted on a back side of the imaging chip 43, that is, on a surface opposite to a surface (the light receiving surface) where the light receiving unit 43a is formed. Examples of the planar devices 44a-1, 44b-1, and 44c-1 include: a buffer, a condenser, an inductor, and a resistance, which amplify an image signal generated by the imaging chip 43 and output the amplified image signal to the signal cables 48; and a processing circuit that processes the image signal, such as a noise removal circuit that removes noise in the image signal generated by the imaging chip 43, or an analog-digital conversion circuit that converts the image signal from an analog signal to a digital signal. By the semiconductor chips 44a, 44b, and 44c being layered over one another and mounted on the imaging chip 43, a rigid portion of the imaging unit 40 in the optical axis direction is able to be decreased in length.

Each of the semiconductor chips 44a, 44b, and 44c has a through-via 54, formed therein. The semiconductor chips 44a, 44b, and 44c are connected, via the through-vias 54 and the bump 53 and bumps 53 to the semiconductor chips and the imaging chip 43 adjacent thereto. Further, sizes of the semiconductor chips 44a, 44b, and 44c projected onto a plane orthogonal to the optical axis direction is a size fitting into a plane of optical axis direction projection of the imaging chip 43.

The FPC board 45 is connected to the semiconductor chip 44c via a bump 53, and has a bent portion not contacting the semiconductor chip. Core wires 48a of the signal cables 48 are connected to the bent portion of the FPC board 45.

The reinforcement member 46 is a cylindrical frame, and is formed of metal. The reinforcement member 46 is, as illustrated in FIG. 3B, arranged to cover outer peripheral side surfaces of the semiconductor chips 44a, 44b, and 44c. By the reinforcement member 46 being arranged to cover a connection interface between the semiconductor chips 44a and 44b, and a connection interface between the semiconductor chips 44b and 44c, durability against stress in a shearing direction is able to be improved. Further, by the sizes of the semiconductor chips 44a, 44b, and 44c projected onto the plane orthogonal to the optical axis direction being made smaller than the size of the imaging chip 43, sizes of the reinforcement member 46 and the cover glass 49 projected onto the surface orthogonal to the optical axis direction are made sizes fitting into the plane of optical axis direction projection of the imaging chip 43. Thereby, a radial direction length of the imaging unit 40, that is, a length thereof in a direction orthogonal to the optical axis direction, is able to be maintained.

A sealing resin 55 is injected in a connected portion between the imaging chip 43 and the semiconductor chip 44a, a connected portion between the semiconductor chips 44a and 44b, and a connected portion between the semiconductor chips 44b and 44c, thereby improving connection strength.

Further, the sealing resin 55 is also injected between the reinforcement member 46 and the semiconductor chips 44a, 44b, and 44c, thereby fixing the reinforcement member 46 thereto, and insulating between the reinforcement member 46 and the semiconductor chips 44a, 44b, and 44c.

As described above, according to the first embodiment of the present invention, since the connection interfaces among the semiconductor chips 44a, 44b, and 44c are protected by the reinforcement member 46 while the semiconductor chips 44a, 44b, and 44c are layered over one another and mounted on a back surface of the imaging chip 43, the imaging unit 40 having a rigid portion that is short and excellent in reliability is able to be obtained.

In this first embodiment, the reinforcement member 46 that is made of metal is used, but the reinforcement member 46 may be made of resin. Since a molded reinforcement member made of resin is difficult to be manufactured in terms of downsizing and thickness, by coating of the outer peripheral side surfaces of the semiconductor chips 44a, 44b, and 44c with resin, the strength against stress in the shearing direction is able to be improved. Further, the resin to be used is preferably high in moisture resistance.

Further, in this first embodiment, connection between the imaging chip 43 and the semiconductor chip 44a, or between the semiconductor chips is achieved via the bump 53, but not being limited thereto, an oxide film formed on a surface of the imaging chip 43 or semiconductor chips may be directly connected by pressure or the like, without the provision of the bump 53.

Further, a protective layer made of resin may be formed beforehand on an outer peripheral portion of the back surface of the imaging chip 43, the outer peripheral portion being a contacting portion between the imaging chip 43 and the reinforcement member 46. By this formation of the protective layer, chippage of the imaging chip 43 due to contact between the reinforcement member 46 and the imaging chip 43 is able to be prevented.

Further, although the planar devices 44a-1, 44b-1, and 44c-1 are formed only on one side of the semiconductor chips 44a, 44b, and 44c, semiconductor chips having planar devices on both sides may be used. Furthermore, the number of semiconductor chips used may be two or more, and is not limited to three.

Further, in the first embodiment, the signal cables 48 are connected via the FPC board 45, but end faces of the signal cables 48 may be directly connected to the semiconductor chip 44c.

### (First Modified Example of First Embodiment)

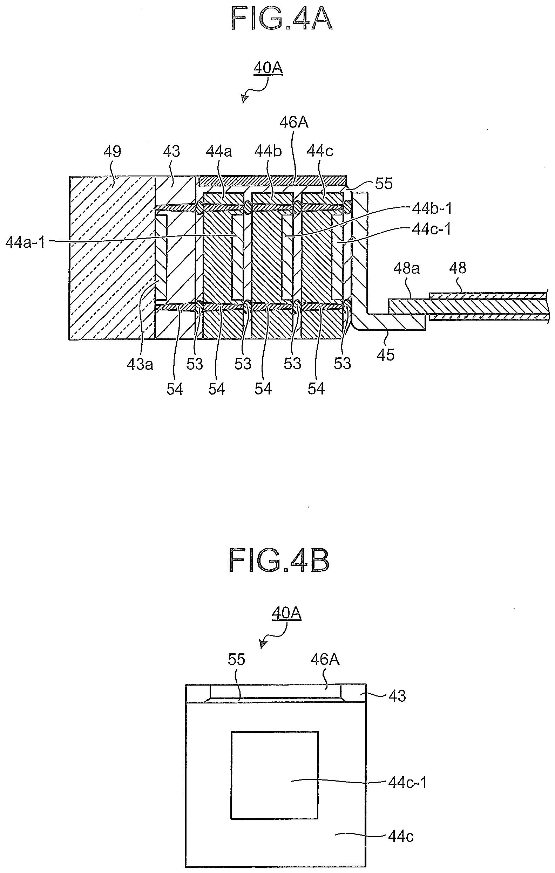

FIG. 4A is a sectional view of an imaging unit according a first modified example of the first embodiment. FIG. 4B is a rear view of the imaging unit according to the first modified example of the first embodiment, and for understanding of the invention, illustration of the FPC board 45 is omitted therein.

An imaging unit 40A includes a reinforcement member 46A that is plate-like. The reinforcement member 46A is arranged to be in contact with one of side surfaces of the semiconductor chips 44a, 44b, and 44c, and is connected by the sealing resin 55, to cover the connection interface between the semiconductor chips 44a and 44b and the connection interface between the semiconductor chips 44b and 44c.

By the reinforcement member 46A being arranged to cover the connection interfaces between the semiconductor chips 44a and 44b and between the semiconductor chips 44b and 44c, the strength against stress in the shearing direction is able to be improved. Further, since the semiconductor chips 44a, 44b, and 44c are layered over one another and mounted on the back surface of the imaging chip 43, and the cover glass 49, the reinforcement member 46A, and the semiconductor chips 44a, 44b, and 44c are arranged to fit into the plane of optical axis direction projection of the imaging chip 43; the imaging unit 40A is able to be downsized.

### (Second Modified Example of First Embodiment)

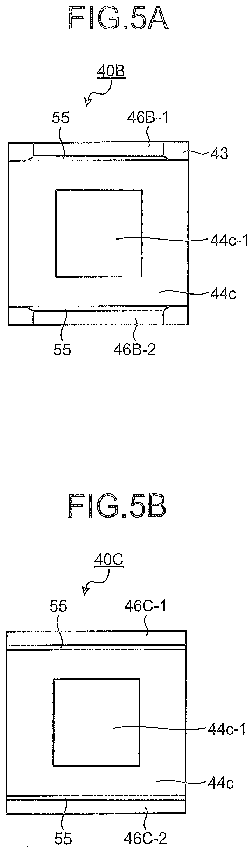

FIG. 5A is a rear view of an imaging unit according to a second modified example of the first embodiment, and for understanding of the invention, illustration of the FPC board 45 is omitted therein.

An imaging unit 40B includes two reinforcement members 46B-1 and 46B-2 that are plate-like. The reinforcement members 46B-1 and 46B-2 are arranged to be in contact with two opposite side surfaces of the semiconductor chips 44a, 44b, and 44c, and are connected by the sealing resin 55, to cover the connection interface between the semiconductor chips 44a and 44b and the connection interface between the semiconductor chips 44b and 44c.

By the reinforcement members 46B-1 and 46B-2 being arranged to cover the connection interfaces between the semiconductor chips 44a and 44b and between the semiconductor chips 44b and 44c, strength against stress in the shearing direction is able to be improved. Further, since the semiconductor chips 44a, 44b, and 44c are layered over one another and mounted on the back surface of the imaging chip 43, and the cover glass 49, the reinforcement members 46B-1 and 46B-2, and the semiconductor chips 44a, 44b, and 44c are arranged to fit into the plane of optical axis direction projection of the imaging chip 43; the imaging unit 40B is able to be downsized.

### (Third Modified Example of First Embodiment)

FIG. 5B is a rear view of an imaging unit according to a third modified example of the first embodiment, and for understanding of the invention, illustration of the FPC board 45 is omitted therein.

An imaging unit 40C includes two reinforcement members 46C-1 and 46C-2 that are plate-like. Lengths of the reinforcement members 46C-1 and 46C-2 in a direction orthogonal to the optical axis direction are longer than lengths of the reinforcement members 46B-1 and 46B-2, and are substantially the same as lengths of the semiconductor chips 44a, 44b, and 44c. The reinforcement members 46C-1 and 46C-2 are arranged to be in contact with the two opposite side surfaces of the semiconductor chips 44a, 44b, and 44c, and are connected by the sealing resin 55, to cover the connection interface between the semiconductor chips 44a and 44b and the connection interface between the semiconductor chips 44b and 44c.

By the reinforcement members 46C-1 and 46C-2 being arranged to cover the connection interfaces between the semiconductor chips 44a and 44b and between the semiconductor chips 44b and 44c, strength against stress in the shearing direction is able to be improved. Further, since the semiconductor chips 44a, 44b, and 44c are layered over one another and mounted on the back surface of the imaging chip 43, and the cover glass 49, the reinforcement members 46C-1 and 46C-2, and the semiconductor chips 44a, 44b, and 44c are arranged to fit into the plane of optical axis direction projection of the imaging chip 43; the imaging unit 40C is able to be downsized.

### (Fourth Modified Example of First Embodiment)

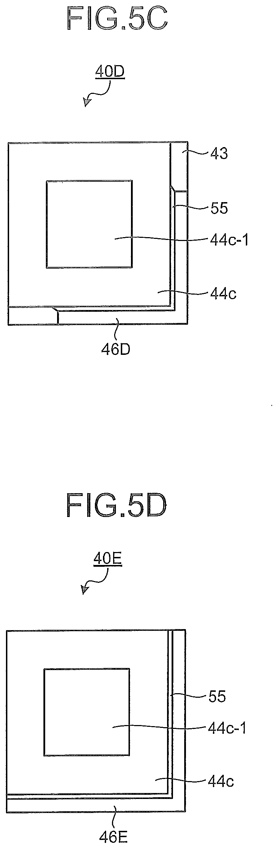

FIG. 5C is a rear view of an imaging unit according to a fourth modified example of the first embodiment, and for understanding of the invention, illustration of the FPC board 45 is omitted therein.

An imaging unit 40D includes a reinforcement member 46D having an L-shaped cross section. The reinforcement member 46D is arranged to be in contact with two adjacent side surfaces of the semiconductor chips 44a, 44b, and 44c, and is connected by the sealing resin 55, to cover the connection interface between the semiconductor chips 44a and 44b and the connection interface between the semiconductor chips 44b and 44c.

By the reinforcement member 46D being arranged to cover the connection interfaces between the semiconductor chips 44a and 44b and between the semiconductor chips 44b and 44c, strength against stress in the shearing direction is able to be improved. Further, since the semiconductor chips 44a, 44b, and 44c are layered over one another and mounted on the back surface of the imaging chip 43, and the cover glass 49, the reinforcement member 46D, and the semiconductor chips 44a, 44b, and 44c are arranged to fit into the plane of optical axis direction projection of the imaging chip 43; the imaging unit 40D is able to be downsized.

### (Fifth Modified Example of First Embodiment)

FIG. 5D is a rear view of an imaging unit according to a fifth modified example of the first embodiment, and for understanding of the invention, illustration of the FPC board 45 is omitted therein.

An imaging unit 40E includes a reinforcement member 46E having an L-shaped cross section. A length of each side of the reinforcement member 46E in a direction orthogonal to the optical axis direction is longer than a length of each side of the reinforcement member 46D, and is substantially the same as a length of each side of the semiconductor chips 44a, 44b, and 44c. The reinforcement member 46E is arranged to be in contact with two adjacent side surfaces of the semiconductor chips 44a, 44b, and 44c, and is connected by the sealing resin 55, to cover the connection interface between the semiconductor chips 44a and 44b and the connection interface between the semiconductor chips 44b and 44c.

By the reinforcement member 46E being arranged to cover the connection interfaces between the semiconductor chips 44a and 44b and between the semiconductor chips 44b and 44c, strength against stress in the shearing direction is able to be improved. Further, since the semiconductor chips 44a, 44b, and 44c are layered over one another and mounted on the back surface of the imaging chip 43, and the cover glass 49, the reinforcement member 46E, and the semiconductor chips 44a, 44b, and 44c are arranged to fit into the plane of optical axis direction projection of the imaging chip 43; the imaging unit 40E is able to be downsized.

### (Sixth Modified Example of First Embodiment)

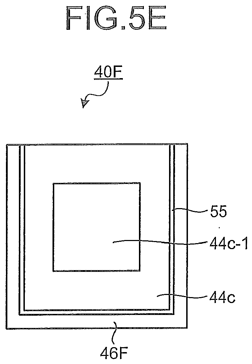

FIG. 5E is a rear view of an imaging unit according to a sixth modified example of the first embodiment, and for understanding of the invention, illustration of the FPC board 45 is omitted therein.

An imaging unit 40F includes a reinforcement member 46F having a U-shaped cross section. The reinforcement member 46F is arranged to be in contact with three side surfaces of the semiconductor chips 44a, 44b, and 44c, and is connected by the sealing resin 55, to cover the connection interface between the semiconductor chips 44a and 44b and the connection interface between the semiconductor chips 44b and 44c.

By the reinforcement member 46F being arranged to cover the connection interfaces between the semiconductor chips 44a and 44b, and the connection interface between the semiconductor chips 44b and 44c, strength against stress in the shearing direction is able to be improved. Further, since the semiconductor chips 44a, 44b, and 44c are layered over one another and mounted on the back surface of the imaging chip 43, and the cover glass 49, the reinforcement member 46F, and the semiconductor chips 44a, 44b, and 44c are arranged to fit into the plane of optical axis direction projection of the imaging chip 43; the imaging unit 40F is able to be downsized.

### (Seventh Modified Example of First Embodiment)

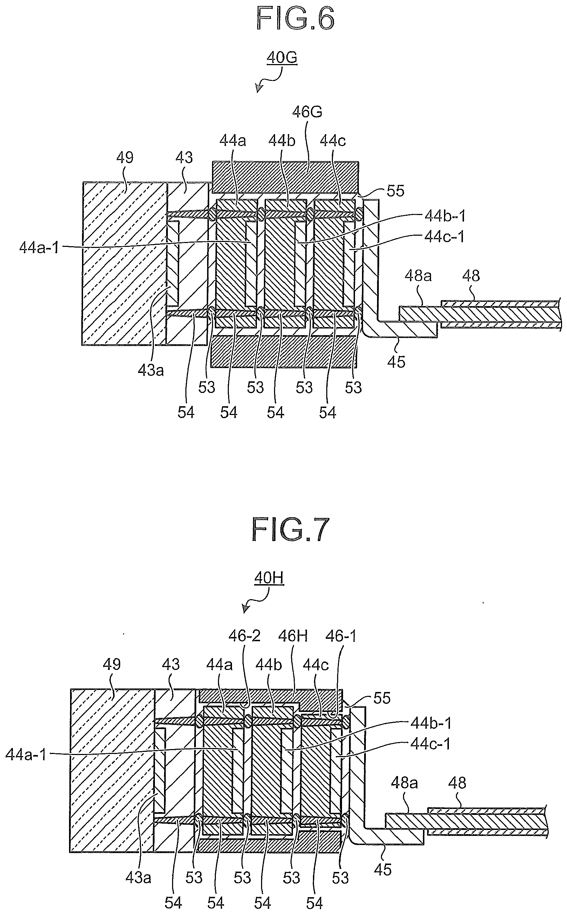

FIG. 6 is a sectional view of an imaging unit according a seventh modified example of the first embodiment.

An imaging unit 40G includes a reinforcement member 46G that forms a cylindrical frame. The reinforcement member 46G is arranged to be in contact with three side surfaces of the semiconductor chips 44a, 44b, and 44c, and is connected by the sealing resin 55, to cover the connection interface between the semiconductor chips 44a and 44b and the connection interface between the semiconductor chips 44b and 44c.

By the reinforcement member 46G being arranged to cover the connection interfaces between the semiconductor chips 44a and 44b and between the semiconductor chips 44b and 44c, strength against stress in the shearing direction is able to be improved. Further, since the semiconductor chips 44a, 44b, and 44c are layered over one another and mounted on the back surface of the imaging chip 43, a length of the imaging unit 40G in the optical axis direction is able to be decreased. Although the reinforcement member 46G is larger than the plane of optical axis direction projection of the imaging chip 43, since the semiconductor chips 44a, 44b, and 44c are smaller than the plane of optical axis direction projection of the imaging chip 43, increase in radial direction length of the imaging unit 40G is able to be kept low.

### (Eighth Modified Example of First Embodiment)

FIG. 7 is a sectional view of an imaging unit according an eighth modified example of the first embodiment.

An imaging unit 40H includes a reinforcement member 46H, which forms a cylindrical frame, and which has a small diameter portion 46-1 and a large diameter portion 46-2 therein. As to sizes of the semiconductor chips 44a, 44b, and 44c in the optical axis direction, the sizes of the semiconductor chips 44a and 44b are the same, and the semiconductor chip 44c is smaller than the semiconductor chips 44a and 44b. The reinforcement member 46H is formed, such that when the reinforcement member 46H is arranged on the outer peripheral side surfaces of the semiconductor chips 44a, 44b, and 44c, an inner periphery of the small diameter portion 46-1 contacts the semiconductor chip 44c and an inner periphery of the large diameter portion 46-2 contacts the semiconductor chips 44a and 44b. The reinforcement member 46H is connected to the semiconductor chips 44a, 44b, and 44c by the sealing resin 55, to cover the connection interface between the semiconductor chips 44a and 44b and the connection interface between the semiconductor chips 44b and 44c.

By the reinforcement member 46H being arranged to cover the connection interfaces between the semiconductor chips 44a and 44b and between the semiconductor chips 44b and 44c, strength against stress in the shearing direction is able to be improved. Further, since the semiconductor chips 44a, 44b, and 44c are layered over one another and mounted on the back surface of the imaging chip 43, and the cover glass 49, the reinforcement member 46H, and the semiconductor chips 44a, 44b, and 44c are arranged to fit into the plane of optical axis direction projection of the imaging chip 43; the imaging unit 40H is able to be downsized.

### (Second Embodiment)

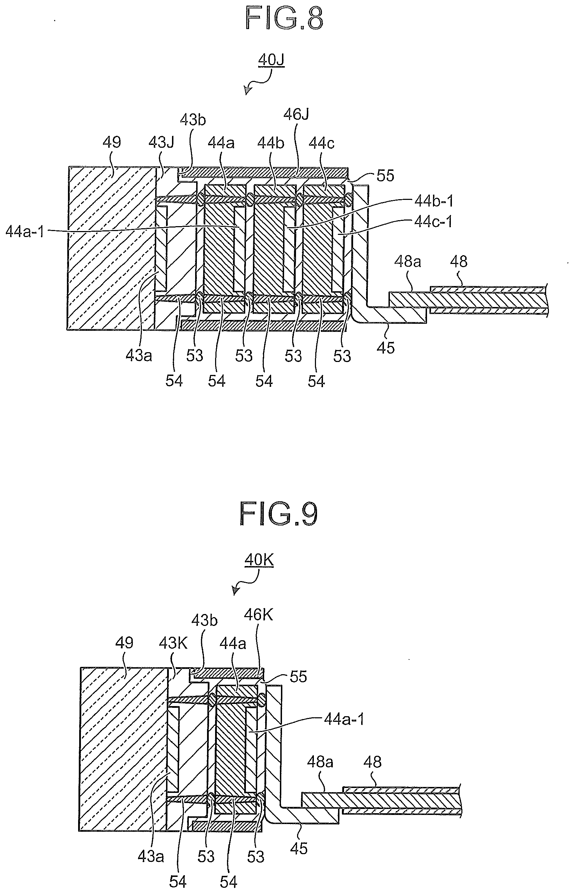

FIG. 8 is a sectional view of an imaging unit according to a second embodiment. An imaging unit 40J according to the second embodiment includes an imaging chip 43J having a first stepped portion 43b on a back side thereof.

The first stepped portion 43b is provided over the whole outer periphery of a back surface of the imaging chip 43J, and a reinforcement member 46J is arranged such that a cylindrical end portion of the reinforcement member 46J contacts this first stepped portion 43b. Further, the reinforcement member 46J is arranged in contact with side surfaces of the first stepped portion 45b and semiconductor chips 44a, 44b, and 44c, and are connected by the sealing resin 55 to cover a connection interface between the imaging chip 43J and the semiconductor chip 44a, the connection interface between the semiconductor chips 44a and 44b, and the connection interface between the semiconductor chips 44b and 44c.

By the reinforcement member 46J being arranged to cover the connection interfaces between the imaging chip 43J and the semiconductor chip 44a, between the semiconductor chips 44a and 44b, and between the semiconductor chips 44b and 44c, strength against stress in the shearing direction is able to be improved. Further, since the semiconductor chips 44a, 44b, and 44c are layered over one another and mounted on the back surface of the imaging chip 43J, and the cover glass 49, the reinforcement member 46J, and the semiconductor chips 44a, 44b, and 44c are arranged to fit into a plane of optical axis direction projection of the imaging chip 43J; the imaging unit 40J is able to be downsized. In other words, by the sizes of the semiconductor chips 44a, 44b, and 44c projected onto the plane orthogonal to the optical axis direction being made smaller than a size of the imaging chip 43J, and the first stepped portion 43b being provided in the imaging chip 43J; a size of the reinforcement member 46J projected onto the plane orthogonal to the optical axis direction is able to be made a size fitting into the plane of optical axis direction projection of the imaging chip 43J, and downsizing is enabled. Furthermore, a protective layer made of resin may be formed beforehand on a bottom surface of the first stepped portion 43b provided on the outer periphery of the back surface of the imaging chip 43J, the bottom surface being a contacting portion between the imaging chip 43J and the reinforcement member 46J. By this formation of the protective layer, chippage of the imaging chip 43J due to contact between the reinforcement member 46J and the imaging chip 43J is able to be prevented.

### (First Modified Example of Second Embodiment)

FIG. 9 is a sectional view of an imaging unit according to a first modified example of the second embodiment. An imaging unit 40K according to the first modified example of the second embodiment includes, similarly to the second embodiment, an imaging chip 43K having the first stepped portion 43b on a back side thereof, and only the semiconductor chip 44a is mounted on a back surface of the imaging chip 43K.

A reinforcement member 46K is arranged in contact with the side surfaces of the first stepped portion 43b and the semiconductor chip 44a, and is connected by the sealing resin 55, to cover a connection interface between the imaging chip 43K and the semiconductor chip 44a.

By the reinforcement member 46K being arranged to cover the connection interface between the imaging chip 43K and the semiconductor chip 44a, strength against stress in the shearing direction is able to be improved. Further, since the semiconductor chip 44a is mounted on the back surface of the imaging chip 43K, and the cover glass 49, the reinforcement member 46K, and the semiconductor chip 44a are arranged to fit into a plane of optical axis direction projection of the imaging chip 43K, the imaging unit 40K is able to be downsized.

### (Third Embodiment)

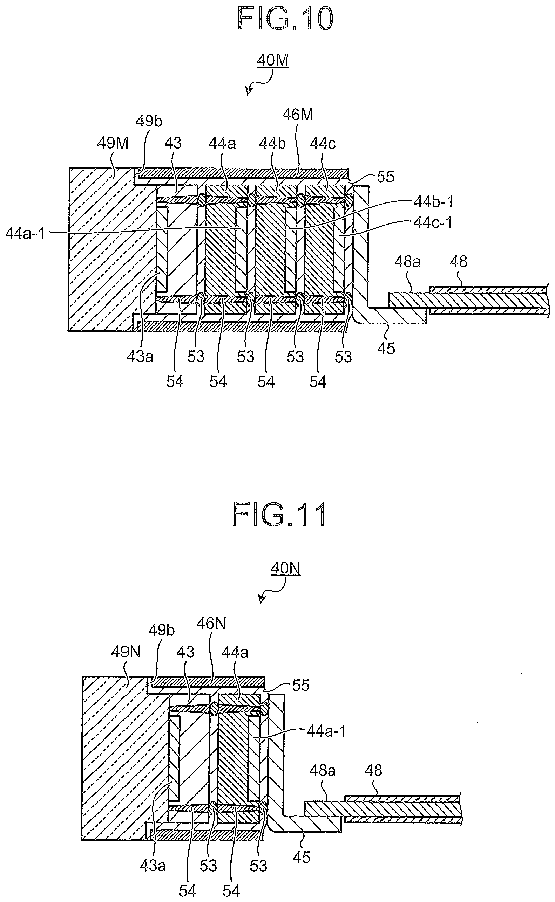

FIG. 10 is a sectional view of an imaging unit according to a third embodiment. An imaging unit 40M according to the third embodiment includes a cover glass 49M having a second stepped portion 49b on a back side thereof.

The second stepped portion 49b is provided over the whole outer periphery of a back surface of the cover glass 49M, and a reinforcement member 46M is arranged such that a cylindrical end portion of the reinforcement member 46M contacts this second stepped portion 49b. Further, the reinforcement member 46M is arranged to be in contact with side surfaces of the second stepped portion 49b, the imaging chip 43, and the semiconductor chips 44a, 44b, and 44c, and is connected by the sealing resin 55, to cover a connection interface between the cover glass 49M and the imaging chip 43, the connection interface between the imaging chip 43 and the semiconductor chip 44a, the connection interface between the semiconductor chips 44a and 44b, and the connection interface between the semiconductor chips 44b and 44c.

By the reinforcement member 46M being arranged to cover the connection interfaces between the cover glass 49M and the imaging chip 43, between the imaging chip 43 and the semiconductor chip 44a, between the semiconductor chips 44a and 44b, and between the semiconductor chips 44b and 44c, strength against stress in the shearing direction is able to be improved even more. Further, since the semiconductor chips 44a, 44b, and 44c are layered over one another and mounted on the back surface of the imaging chip 43, and the imaging chip 43, the reinforcement member 46M, and the semiconductor chips 44a, 44b, and 44c are arranged to fit into a plane of optical axis direction projection of the cover glass 49M; the imaging unit 40M is able to be downsized. In other words, by sizes of the imaging chip 43 and the semiconductor chips 44a, 44b, and 44c projected onto a plane orthogonal to the optical axis direction being made smaller than a size of the cover glass 49M, and the second stepped portion 49b being provided in the cover glass 49M; a size of the reinforcement member 46M projected onto the plane orthogonal to the optical direction is able to be made a size fitting into the size of the plane of optical axis direction projection of the cover glass 49M, and downsizing is enabled.

Furthermore, a protective layer made of resin may be formed beforehand on a bottom surface of the second stepped portion 49b provided on the outer periphery of the back surface of the cover glass 49M, the bottom surface being a contacting portion between the cover glass 49M and the reinforcement member 46M. By this formation of the protective layer, chippage of the cover glass 49M due to contact between the reinforcement member 46M and the cover glass 49M is able to be prevented.

### (First Modified Example of Third Embodiment)

FIG. 11 is a sectional view of an imaging unit according to a first modified example of the third embodiment. An imaging unit 40N according to the first modified example of the third embodiment includes a cover glass 49N having a second stepped portion 49b on a back side thereof, and has only the semiconductor chip 44a mounted on the back surface of the imaging chip 43.

The second stepped portion 49b is provided over the whole outer periphery of a back surface of the cover glass 49N, and a reinforcement member 46N is arranged such that a cylindrical end portion of the reinforcement member 46N contacts this second stepped portion 49b. Further, the reinforcement member 46N is arranged in contact with side surfaces of the second stepped portion 49b, imaging chip 43, and semiconductor chip 44a, and is connected by the sealing resin 55, to cover a connection interface between the cover glass 49N and the imaging chip 43, and the connection interface between the imaging chip 43 and the semiconductor chip 44a.

By the reinforcement member 46N being arranged to cover the connection interfaces between the cover glass 49N and the imaging chip 43 and between the imaging chip 43 and the semiconductor chip 44a, strength against stress in the shearing direction is able to be improved even more. Furthermore, since the semiconductor chip 44a is layered and mounted on the back surface of the imaging chip 43, and the imaging chip 43, the reinforcement member 46N, and the semiconductor chip 44a are arranged to fit into a plane of optical axis direction projection of the cover glass 49N, the imaging unit 40N is able to be downsized.

### (Second Modified Example of Third Embodiment)

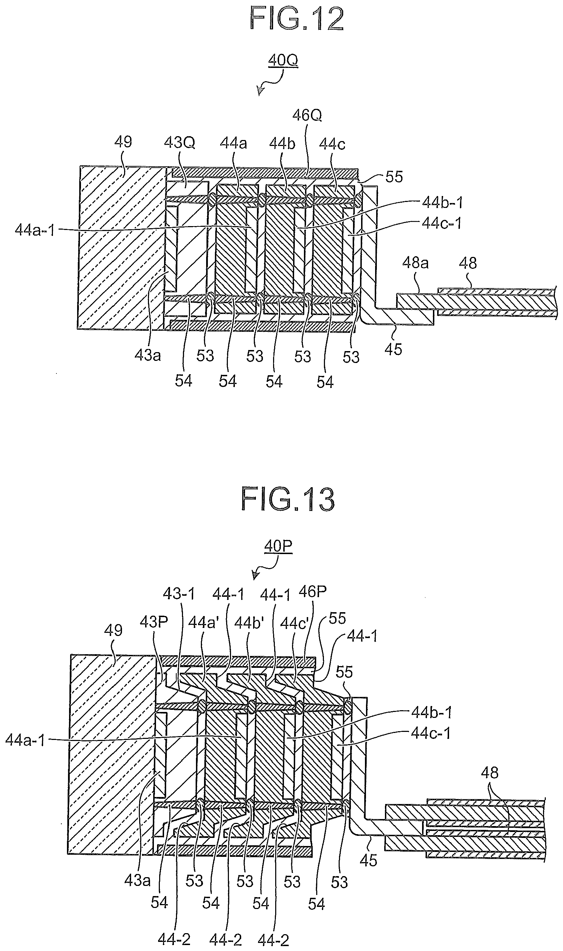

FIG. 12 is a sectional view of an imaging unit according to a second modified example of the third embodiment.

In an imaging unit 40Q according to the second modified example of the third embodiment, a reinforcement member 46Q that is cylindrical is arranged in contact with side surfaces of an imaging chip 43Q, and the semiconductor chips 44a, 44b, and 44c, and an end portion of the reinforcement member 46Q contacts a back surface of the cover glass 49. The reinforcement member 46Q is connected by the sealing resin 55, to cover a connection interface between the imaging chip 43Q and the semiconductor chip 44a, the connection interface between the semiconductor chips 44a and 44b, and the connection interface between the semiconductor chips 44b and 44c.

By the reinforcement member 46Q being arranged to cover the connection interfaces between the imaging chip 43Q and the semiconductor chip 44a, between the semiconductor chips 44a and 44b, and between the semiconductor chips 44b and 44c, strength against stress in the shearing direction is able to be improved. Furthermore, since the semiconductor chips 44a, 44b, and 44c are layered over one another and mounted on the back surface of the imaging chip 43Q, and the imaging chip 43Q, the reinforcement member 46Q, and the semiconductor chips 44a, 44b, and 44c are arranged to fit into a plane of optical axis direction projection of the cover glass 49; the imaging unit 40Q is able to be downsized.

### (Third Modified Example of Third Embodiment)

FIG. 13 is a sectional view of an imaging unit according to a third modified example of the third embodiment.

An imaging unit 40P according to the third modified example of the third embodiment includes an imaging chip 43P having a recessed portion 43-1 formed over the whole outer periphery of a back surface of the imaging chip 43P. Further, each of semiconductor chips 44a', 44b', and 44c' layered over one another and mounted on a back side of the imaging chip 43P has, on a front side thereof, a protruded portion 44-2 fittable to the recessed portion 43-1 of the imaging chip 43P, and on a back side thereof, a recessed portion 44-1 (having the same shape as the recessed portion 43-1) fittable to the recessed portion 43-1. The recessed portions 43-1 and 44-1 and the protruded portions 44-2 may be formed by etching, for example, crystal anisotropic wet etching, or ICP taper etching. Connection between the imaging chip 43P and the semiconductor chip 44a', between the semiconductor chips 44a' and 44b', and between the semiconductor chips 44b' and 44c', is achieved by fitting between the recessed portion 43-1 and the protruded portion 44-2, and between the recessed portions 44-1 and the protruded portions 44-2. Connection electrodes may be formed on inclined surfaces and bottom surfaces of the recessed portions 43-1 and 44-1 and the protruded portions 44-2.

A reinforcement member 46P that is cylindrical is arranged in contact with side surfaces of the imaging chip 43P and the semiconductor chips 44a', 44b', and 44c', and an end portion of the reinforcement member 46P contacts the back surface of the cover glass 49. The reinforcement member 46P is arranged to cover a connection interface between the imaging chip 43P and the semiconductor chip 44a', a connection interface between the semiconductor chips 44a' and 44b', and a connection interface between the semiconductor chips 44b' and 44c', and is connected by the sealing resin 55.

By the arrangement of the reinforcement member 46P to cover the connection interfaces between the imaging chip 43P and the semiconductor chip 44a', between the semiconductor chips 44a' and 44b', and between the semiconductor chips 44b' and 44c', strength against stress in the shearing direction is able to be improved. Further, since the connection between the imaging chip 43P and the semiconductor chip 44a', between the semiconductor chips 44a' and 44b', and between the semiconductor chips 44b' and 44c', is achieved by fitting between the recessed portion 43-1 and the protruded portion 44-2, and between the recessed portions 44-1 and the protruded portions 44-2, connection strength is able to be improved even more. Furthermore, since the semiconductor chips 44a', 44b', and 44c' are layered over one another and mounted on the back surface of the imaging chip 43P, and the imaging chip 43P, the reinforcement member 46P, and the semiconductor chips 44a', 44b', and 44c' are arranged to fit into the plane of optical axis direction projection of the cover glass 49, the imaging unit 40P is able to be downsized.

In the above described third modified example of the third embodiment, the signal cables 48 are connected via the FPC board 45, but a molded interconnect device (MID) having a recessed portion fittable to the protruded portion 44-2 of the semiconductor chip 44c' may be used instead of the FPC board 45.

### Reference Signs List

1 ENDOSCOPE SYSTEM
2 ENDOSCOPE
3 UNIVERSAL CORD
3A DISTAL END PORTION
3B BENDING PORTION
3C FLEXIBLE TUBE PORTION
4 OPERATING UNIT
4a TREATMENT TOOL INSERTION OPENING
4b BENDING OPERATION KNOB
5 CONNECTOR PORTION
6 PROCESSOR
7 DISPLAY DEVICE
8 LIGHT SOURCE DEVICE
30 INSERTION PORTION
35 IMAGING DEVICE
36 LENS UNIT
36a-1, 36a-2, 36a-3, 36a-4 OBJECTIVE LENS
36b LENS HOLDER
40, 40A, 40B, 40C, 40D, 40E, 40F, 40G, 40H, 40J, 40K, 40M, 40N, 40P, 40Q IMAGING UNIT
41 DISTAL END PORTION MAIN BODY
41a ADHESIVE
42 COVERING TUBE
43, 43J, 43K, 43P, 43Q IMAGING CHIP
43a LIGHT RECEIVING UNIT
43-1, 44-1 RECESSED PORTION
43b FIRST STEPPED PORTION
44a, 44b, 44c, 44a', 44b', 44c' SEMICONDUCTOR CHIP
44a-1, 44b-1, 44c-1 PLANAR DEVICE
44-2 PROTRUDED PORTION
45 FLEXIBLE PRINTED CIRCUIT BOARD
46, 46A, 46B-1, 46B-2, 46C-1, 46C-2, 46D, 46E, 46F, 46G, 46H, 46J, 46K, 46M, 46N, 46P, 46Q REINFORCEMENT MEMBER
47 ELECTRIC CABLE BUNDLE
48 SIGNAL CABLE
48a CORE WIRE
49 COVER GLASS
49b SECOND STEPPED PORTION
50 HEAT SHRINKABLE TUBE
51, 55 SEALING RESIN
53 BUMP
54 THROUGH-VIA
81 BENDING TUBE
82 BENDING WIRE

## Claims

1. An imaging unit comprising:
an imaging chip configured to generate an image signal by receiving light via a light receiving surface thereof and performing photoelectric conversion;
at least one semiconductor chip having a size fitting into a plane of projection of the imaging chip, the size being projected onto a plane orthogonal to an optical axis direction; and
a reinforcement member arranged on at least one side surface of the semiconductor chip, wherein
the semiconductor chip is layered and mounted on a back side of the light receiving surface of the imaging chip, and the reinforcement member is arranged to cover a connection interface between the imaging chip and the semiconductor chip, or a connection interface between the semiconductor chips.

2. The imaging unit according to claim 1, wherein the reinforcement member has a cross section having a shape of an L-shaped plate, the cross section being orthogonal to the optical axis direction, and the reinforcement member is arranged to cover two sides of the semiconductor chip, or two sides of the imaging chip and the semiconductor chip.

3. The imaging unit according to claim 1, wherein the reinforcement member is cylindrical, and is arranged on an outer peripheral side surface of the semiconductor chip, or an outer peripheral side surface of the imaging chip and the semiconductor chip.

4. The imaging unit according to claim 1, wherein
the imaging chip includes a first stepped portion on a back side of the light receiving surface, and
the reinforcement member is cylindrical, and is arranged on an outer peripheral side surface of the first stepped portion and the semiconductor chip.

5. The imaging unit according to claim 4, wherein a size of the reinforcement member projected onto the plane orthogonal to the optical axis direction is a size fitting into the plane of optical axis direction projection of the imaging chip.

6. The imaging unit according to claim 1, comprising:
a cover glass configured to cover a light receiving unit of the imaging chip, wherein
the cover glass includes a second stepped portion on a surface thereof that contacts the imaging chip, and
the reinforcement member is cylindrical, and is arranged on an outer peripheral side surface of the second stepped portion, the imaging chip, and the semiconductor chip/chips.

7. The imaging unit according to claim 6, wherein a size of the reinforcement member projected onto the plane orthogonal to the optical axis direction is a size fitting into a plane of optical axis direction projection of the cover glass.

8. The imaging unit according to any one of claims 1 to 7, wherein the imaging chip and the semiconductor chip are electrically and mechanically connected to each other by through-vias formed respectively in the imaging chip and the semiconductor chip.

9. An endoscope comprising:
the imaging unit according to any one of claims 1 to 8; and
an insertion portion including a distal end portion and being insertable in a subject, the distal end portion being formed of a rigid member and being cylindrical, wherein
the insertion portion includes the imaging unit in an inner space of the distal end portion.

## Amended claims

### Amended claims under Art. 19.1 PCT

1. (Amended) An imaging unit comprising:
an imaging chip configured to generate an image signal by receiving light via a light receiving surface thereof and performing photoelectric conversion;
at least one semiconductor chip having a size fitting into a plane of projection of the imaging chip, the size being projected onto a plane orthogonal to an optical axis direction;
a reinforcement member arranged on at least one side surface of the semiconductor chip; and
a cover glass configured to cover a light receiving unit of the imaging chip, wherein
the semiconductor chip is layered and mounted on a back side of the light receiving surface of the imaging chip, the reinforcement member is arranged to cover a connection interface between the imaging chip and the semiconductor chip, or a connection interface between the semiconductor chips, and an interface between the reinforcement member and the imaging chip and an interface between the reinforcement member and the semiconductor chip are positioned in a plane of optical axis direction projection of the imaging chip or the cover glass.

2. The imaging unit according to claim 1, wherein the reinforcement member has a cross section having a shape of an L-shaped plate, the cross section being orthogonal to the optical axis direction, and the reinforcement member is arranged to cover two sides of the semiconductor chip, or two sides of the imaging chip and the semiconductor chip.

3. The imaging unit according to claim 1, wherein the reinforcement member is cylindrical, and is arranged on an outer peripheral side surface of the semiconductor chip, or an outer peripheral side surface of the imaging chip and the semiconductor chip.

4. The imaging unit according to claim 1, wherein
the imaging chip includes a first stepped portion on a back side of the light receiving surface, and
the reinforcement member is cylindrical, and is arranged on an outer peripheral side surface of the first stepped portion and the semiconductor chip.

5. The imaging unit according to claim 4, wherein a size of the reinforcement member projected onto the plane orthogonal to the optical axis direction is a size fitting into the plane of optical axis direction projection of the imaging chip.

6. (Amended) The imaging unit according to claim 1,
wherein
the cover glass includes a second stepped portion on a surface thereof that contacts the imaging chip, and
the reinforcement member is cylindrical, and is arranged on an outer peripheral side surface of the second stepped portion, the imaging chip, and the semiconductor chip.

7. The imaging unit according to claim 6, wherein a size of the reinforcement member projected onto the plane orthogonal to the optical axis direction is a size fitting into a plane of optical axis direction projection of the cover glass.

8. The imaging unit according to any one of claims 1 to 7, wherein the imaging chip and the semiconductor chip are electrically and mechanically connected to each other by through-vias formed respectively in the imaging chip and the semiconductor chip.

9. An endoscope, comprising:
the imaging unit according to any one of claims 1 to 8; and
an insertion portion including a distal end portion and being insertable in a subject, the distal end portion being formed of a rigid member and being cylindrical, wherein
the insertion portion includes the imaging unit in an inner space of the distal end portion.

Statement under Art. 19.1 PCT
 Claim 1 has been limited to include, in the imaging unit, a cover glass that covers a light receiving unit of the imaging chip, and to clarify that an interface between the reinforcement member and the imaging chip and semiconductor chip/chips is positioned in a plane of optical axis direction projection of the imaging chip or the cover glass (FIG. 3A, FIG. 4A, and FIG. 6 to FIG. 13).

In Claim 6, a limitation related to the cover glass has been deleted because of the addition of the cover glass to Claim 1.
